# FASCICULE DE BREVET EUROPEEN

(11) **EP 2 231 081 B1**
(45) Date de publication et mention de la délivrance du brevet: **20.08.2014**
(21) Numéro de dépôt: 08870652.8
(22) Date de dépôt: 16.10.2008
(51) Int. Cl.: A61F 5/00, A61F 2/00

(54) **ANNEAU GASTRIQUE BI-BALLONNET**
Doppelter Ballon-Magenring
DUAL BALLOON GASTRIC RING

(30) Priorité: 16.10.2007 FR 0707249
(43) Date de publication de la demande: 29.09.2010
(73) Titulaire: Compagnie Europeenne d'Etude et de Recherche de Dispositifs pour l'Implantation par Laparoscopie, 38200 Vienne (FR)
(72) Inventeur: RICOL, Jean-Paul, Gilbert, F-69210 Saint Germain sur l'Arbresle (FR)
(74) Mandataire: Guérin, Jean-Philippe
(86) Numéro de dépôt international: PCT/FR2008/001454
(87) Numéro de publication internationale: WO 2009/090334

(56) Documents cités:
- EP-A- 0 028 962
- WO-A-2005/009305
- WO-A-2008/022360

## Description

### ROMAINE TECHNIQUE

La présente invention se rapporte au domaine technique général des implants chirurgicaux destinés à être implantés dans le corps d'un patient autour d'organe(s) biologique(s) constituant une poche ou un conduit, et plus particulièrement aux anneaux gastriques destinés à former une boucle fermée autour de l'estomac pour réduire le diamètre de l'ouverture du stoma.

La présente invention concerne plus particulièrement un anneau chirurgical implantable destiné à être fermé sur lui-même pour former une boucle close d'axe moyen d'extension (XX') afin d'enserrer un organe biologique constituant une poche ou un conduit en vue de modifier la section de passage dudit organe.

La présente invention concerne également un procédé de fabrication d'un tel anneau chirurgical implantable.

### TECHNIQUE ANTERIEURE

Le document EP0028962 décrit un sphincter artificiel formé d'une bande flexible présentant une série de chambre. Les chambres ont des parois hermétiques flexibles et st interconnectées. Une des chambres est connectée à un conduit à travers lequel on introduit ou retire du fluide au moyen d'une pompe. On réalise ainsi le remplissage ou le vidage des différentes chambres et ainsi leur gonflage et dégonflabe. La bande est disposée autour de l'intestin à proximité du stoma, sur la face extérieure de celui-ci. La pompe est actionnée manuellement.

Lors du gonflement, les chambres se déforment pour occuper un espace intermédiaire entre elles jusqu'à venir en contact.

Il est connu de pratiquer des interventions chirurgicales sur des patients atteints d'obésité sévère qui, en raison de leur surpoids, sont exposés non seulement à une gêne physique, mais également à une charge psychologique ainsi qu'à des maladies annexes, tel que diabète, maladies cardiovasculaires ou encore arthrite sévère.

En particulier, une technique connue consiste à réaliser une constriction gastrique réduisant la taille de l'estomac et par conséquent la prise d'aliments.

A cet effet, on recourt fréquemment à l'utilisation d'anneaux de gastroplastie implantés autour de l'estomac du patient en vue de réduire son volume ainsi que le diamètre de son passage (stoma).

De façon bien connue, de tels anneaux de gastroplastie comportent généralement une bande souple, réalisée en matériau élastomère, et destinée à être fermée vers ses deux extrémités par des moyens de fermeture appropriés afin d'enserrer l'estomac.

En outre, les anneaux connus comportent généralement une chambre de compression annulaire située sur la face interne de la bande souple et dont le volume est réglable par adjonction ou retrait d'un fluide de remplissage. Ainsi, il est possible, à partir d'un anneau de dimensions hors-tout fixes, de régler finement le diamètre interne dudit anneau par une expansion ou une contraction radiale de la chambre.

De tels anneaux implantables donnent généralement satisfaction mais souffrent toutefois d'un certain nombre d'inconvénients, en particulier dus à certaines évolutions incontrôlées de leur position pendant la durée du traitement.

En effet, il s'avère que les anneaux de l'art antérieur sont parfois sujets à des déplacements provoqués par les mouvements naturels de dilatation et de contraction de l'estomac.

Dans certains cas, ces mouvements peuvent même conduire à l'expulsion de l'anneau par glissement ou encore à son retournement sur lui-même, de telle sorte qu'il devient inopérant sur le plan thérapeutique et constitutif d'une gêne pour le patient.

En pareille situation, il est alors nécessaire de procéder à une nouvelle intervention chirurgicale en vue du remplacement ou du repositionnement de l'anneau accidentellement déplacé.

Bien entendu, la réitération de telles opérations chirurgicales correctives mobilise inutilement du personnel médical, cause des désagréments supplémentaires au patient, voire expose ce dernier à des complications postopératoires.

### EXPOSE DE L'INVENTION

Les objets assignés à la présente invention visent par conséquent à porter remède aux inconvénients susmentionnés et à proposer un nouvel anneau chirurgical implantable, notamment gastrique, qui présente une stabilité améliorée garantissant son maintien durable en position fonctionnelle après implantation.

Un autre objet assigné à la présente invention vise à proposer un nouvel anneau chirurgical implantable qui présente un caractère atraumatique et qui soit particulièrement respectueux des tissus qui l'environnent.

Un autre objet assigné à l'invention vise à proposer un nouvel anneau chirurgical qui présente une structure simple et légère.

Un autre objet assigné à la présente invention vise à proposer un nouvel anneau chirurgical dont la fabrication soit simple et peu onéreuse.

Un autre objet assigné à l'invention vise à proposer un nouveau procédé de fabrication d'un anneau chirurgical implantable qui confère audit anneau une excellente stabilité une fois ce dernier implanté.

Enfin, un autre objet assigné à l'invention vise à proposer un nouveau procédé de fabrication d'un anneau chirurgical qui soit simple, peu onéreux et rapide à mettre en oeuvre.

Les objets assignés à l'invention sont atteints à l'aide d'un anneau chirurgical tel que défini dans la revendication 1.

Les objets assignés à l'invention sont également atteints à l'aide d'un procédé de fabrication d'un anneau chirurgical implantable tel que défini dans la revendication 14.

### DESCRIPTIF SOMMAIRE DES DESSINS

D'autres objets, caractéristiques et avantages de l'invention apparaîtront plus en détails à la lecture de la description qui suit, ainsi qu'à l'aide des dessins annexés, donnés à titre purement illustratif et non limitatif, parmi lesquels :
- La figure 1 illustre, selon une vue en perspective, un anneau chirurgical conforme à l'invention dans sa position ouverte.
- La figure 2 illustre, selon une vue en perspective, l'anneau chirurgical de la figure 1 dans sa position fermée.
- La figure 3 illustre, selon une vue schématique en coupe longitudinale, un anneau chirurgical conforme à l'invention placé en position fermée autour d'un organe biologique afin d'enserrer ce dernier.
- La figure 4 illustre, selon une vue en perspective avec arrachement partiel, la variante de réalisation d'un anneau implantable correspondant aux figures 1 et 2.
- La figure 5 illustre, selon une vue en coupe sagittale, la variante de réalisation d'un anneau chirurgical conforme à celle représentée sur les figures 1, 2 et 4.

### MEILLEURE MANIERE DE REALISER L'INVENTION

Dans la description qui suit, il sera fait référence, uniquement à titre d'exemple et par commodité de description, à un anneau de gastroplastie (ou anneau gastrique) conçu pour être implanté autour de l'estomac pour réduire le diamètre de l'ouverture du stoma, ou conçu pour être implanté autour de l'oesophage.

Cependant, l'invention n'est nullement limitée à cette application, et couvre également de manière générale les anneaux chirurgicaux destinés à être implantés dans le corps d'un patient autour d'un organe biologique constituant une poche ou un conduit en vue de modifier la section de passage dudit organe lorsque ce dernier est enserré par l'anneau.

Ainsi, la présente invention peut être adaptée pour le traitement de l'incontinence urinaire ou fécale, ou encore pour la régulation du débit sanguin.

Selon l'application, l'anneau chirurgical sera bien entendu adapté aux dimensions, à l'environnement, et à la sensibilité de l'organe concerné par la constriction, tel que vessie, urètre, intestin, artère, veine, etc.

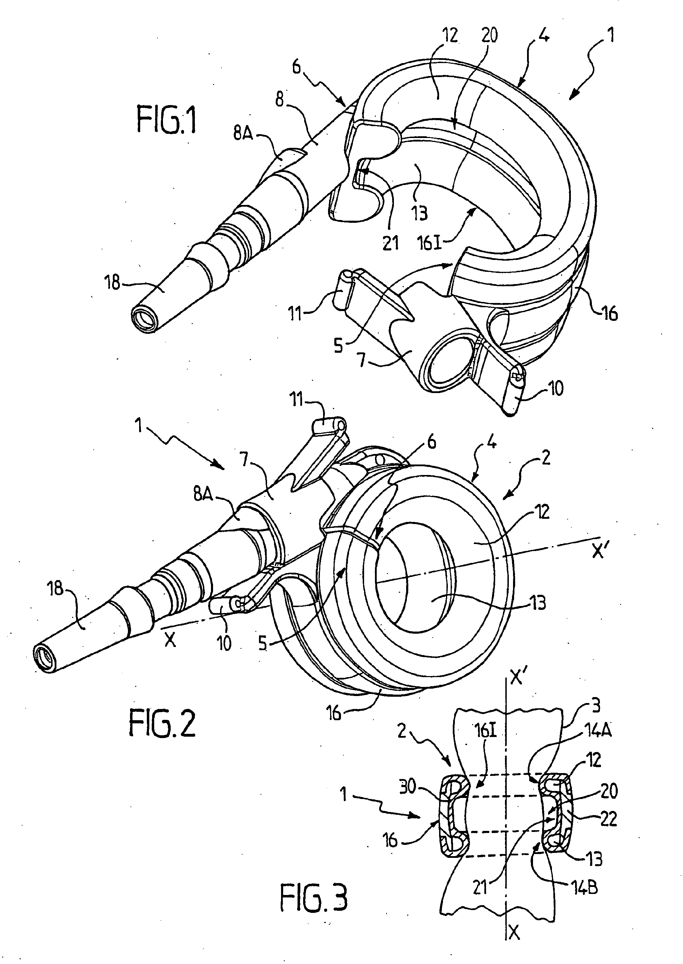

L'anneau chirurgical implantable 1 conforme à l'invention est destiné à être fermé sur lui-même pour former une boucle close 2 d'axe moyen d'extension (XX') afin d'enserrer un organe biologique 3, tel que l'estomac, qui constitue une poche ou un conduit en vue de modifier la section de passage dudit organe, tel que cela est illustré sur la figure 3.

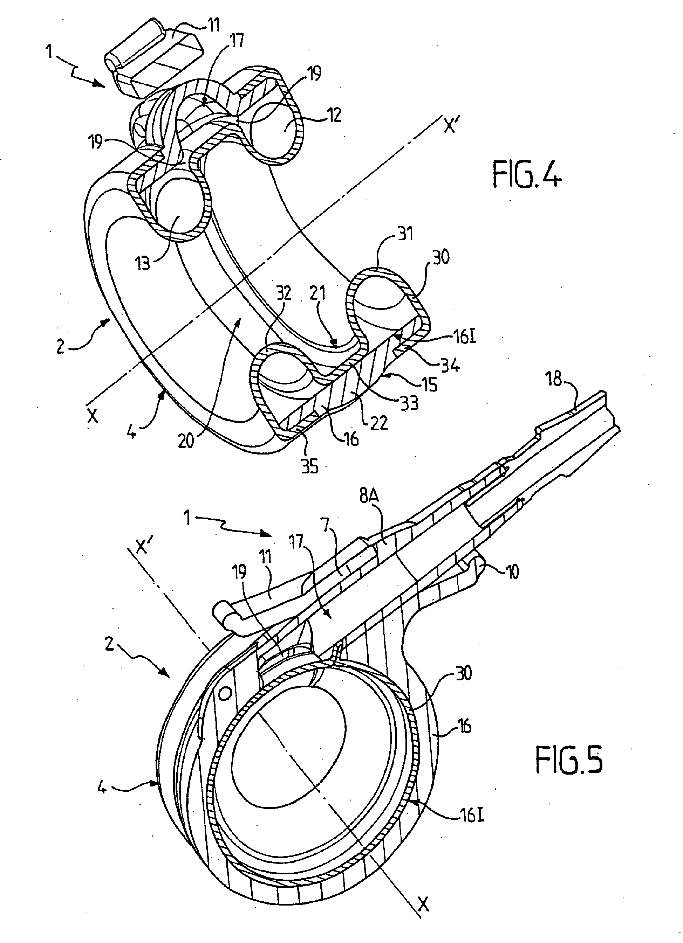

Plus particulièrement, ledit anneau chirurgical 1 possède au moins une portion souple 4 allongée, réalisée de préférence à partir d'un matériau élastomère tel que du silicone, et destinée à passer d'une configuration ouverte, illustrée sur la figure 1 à une configuration fermée, illustrée notamment sur les figures 2 et 5, dans laquelle ladite portion souple est fermée sensiblement vers ses deux extrémités 5, 6 à l'aide de moyens de fermeture 7, 8 appropriés, de sorte à former la boucle close 2 susmentionnée.

Ainsi, en emprisonnant l'organe 3 à l'intérieur de la boucle 2, il est possible de réduire par étranglement le diamètre de sa section de passage, et, dans le cas particulier de l'estomac, l'ouverture du stoma.

Les moyens de fermeture 7, 8 sont conçus pour coopérer entre eux afin d'assurer le verrouillage de l'anneau 1 dans sa configuration fermée. A titre d'exemple, on pourra utiliser à cet effet un manchon 7 fixé sur la première extrémité 5 de la portion souple 4 et destiné à accueillir une tige 8 associée à la seconde extrémité 6, ladite tige 8 comportant de préférence un ou plusieurs ergots 8A de manière à permettre un assemblage de type encliquetage avec le manchon 7.

Bien entendu, la forme adoptée par la boucle close 2 n'est nullement limitée mais présentera de préférence un contour sensiblement arrondi, et préférentiellement sensiblement circulaire.

Ainsi, l'axe moyen d'extension (XX') au sens de l'invention correspond à la ligne génératrice du manchon cylindrique que forme l'anneau chirurgical 1 lorsqu'il se trouve en configuration fermée. Selon une variante de réalisation préférentielle, l'anneau chirurgical 1 forme sensiblement, en position fermée, un cylindre de base circulaire et l'axe moyen d'extension (XX') correspond à la droite rectiligne formant l'axe de révolution dudit anneau.

En outre, l'anneau 1 peut être pourvu d'une ou plusieurs languettes de préhension 10, 11 destinées à faciliter sa manipulation par coelioscopie, notamment lors de sa fermeture et/ou de son ouverture.

Selon une caractéristique importante de l'invention, l'anneau chirurgical 1 comporte au moins une première chambre de serrage 12 annulaire et une seconde chambre de serrage 13 annulaire qui sont étagées l'une par rapport à l'autre le long de l'axe moyen d'extension (XX') de la boucle 2 pour assurer le serrage de l'organe 3 par l'anneau 1 au niveau d'au moins deux zones de contact 14A, 14B séparées axialement.

Ainsi, lesdites première et seconde chambres de serrage 12, 13 assurent avantageusement un double ancrage de l'anneau 1 sur l'organe 3, ce qui améliore la stabilité dudit anneau dans la mesure où, même si ledit anneau venait à perdre prise momentanément au niveau d'un premier point d'ancrage, il subsisterait toujours le second point d'ancrage pour le retenir.

En d'autres termes, les moyens d'ancrage étagés conformes à l'invention permettent de multiplier les points de retenue de l'anneau 1 à l'organe 3, et ainsi de réduire considérablement la probabilité que les conditions cinématiques d'un déplacement dudit anneau par rapport audit organe soient réunies au même instant.

Par conséquent, on limitera sensiblement les phénomènes de déplacement voire de largage de l'anneau.

Afin de fournir un appui à la fois stable, atraumatique, et de préférence réglable, chacune des première et seconde chambres de serrage 12, 13 est destinée à contenir un fluide de remplissage, tel que du sérum physiologique ou encore un gaz tel que l'air.

Bien que lesdites première et/ou seconde chambres de serrage 12, 13 puissent être, selon une variante non représentée, scellées de sorte à contenir un volume de fluide de remplissage fixe prédéterminé par construction, lesdites première et seconde chambres de serrage 12, 13 sont de préférence reliées à un dispositif d'alimentation et de réglage, comprenant par exemple un site implantable disposé sous la peau du patient et permettant le transfert de fluide vers ou depuis lesdites chambres par piquage dudit site à l'aide d'une aiguille creuse.

En outre, la première et/ou la seconde chambre de serrage 12, 13 peuvent être à volume réglable, c'est-à-dire à être délimitées par une membrane élastique susceptible de se déformer en fonction de la pression du fluide de remplissage régnant dans la chambre de serrage, de telle sorte que l'expansion ou la contraction de ladite chambre puisse être réglée par apport ou retrait de fluide de remplissage.

En particulier, la première et la seconde chambre de serrage peuvent avantageusement être agencées de manière à autoriser le réglage fin de l'ouverture du stoma en fonction de leur expansion radiale centripète ou au contraire de leur contraction radiale centrifuge.

Bien entendu, la gestion du fluide de remplissage dans la première et la seconde chambre de serrage 12, 13 est adaptée à la fonction de double ancrage susmentionnée.

Ainsi, selon une caractéristique préférentielle de l'invention, les première et seconde chambres de serrage 12, 13 se trouvent soit en communication fluidique bidirectionnelle libre, soit sensiblement dépourvues de moyen de communication fluidique en elles.

Par *«se frouver en communication fluidique bidirectionnelle libre* », on indique que les première et seconde chambres de serrage 12, 13 communiquent l'une avec l'autre par un ou plusieurs orifices de taille significative et ne faisant pas obstacle à la circulation du fluide de remplissage, de telle sorte que ledit fluide de remplissage peut se déplacer librement de la première vers la seconde chambre, et réciproquement, se déplacer librement de la seconde vers la première chambre.

Selon un tel agencement, la communication franche existant entre les chambres de serrage permet des transferts rapides, voire quasi-instantanés de fluide de remplissage, et par conséquent un équilibrage rapide, voire instantané, des pressions régnant dans lesdites chambres au gré des mouvements naturels de la paroi de l'estomac.

Plus particulièrement, si des fluctuations temporaires de pression exercée par la membrane stomacale sur l'une des chambres de serrage 12, 13 peuvent conduire à un refoulement partiel du fluide contenu dans l'une des chambres de serrage vers l'autre chambre de serrage, et à une augmentation globale de la pression interne régnant dans lesdites chambres 12, 13, le relâchement de la contrainte exercé par la membrane stomacale est rapidement, voire immédiatement, suivi par un retour à l'équilibre du système, de telle sorte que l'effort effectif de serrage de la paroi stomacale par l'anneau chirurgical 1 est maintenu sensiblement en permanence au niveau de chacune des zones de contact 14A, 14B.

Bien que la communication fluidique entre les chambres puisse s'établir par un circuit externe annexe, éventuellement déporté au niveau d'un système d'alimentation commun présentant un débit suffisant, les moyens de communication fluidique bidirectionnelle libre seront de préférence situés au niveau de l'anneau 1 lui-même et intégrés à ce dernier.

Avantageusement, l'anneau 1 peut ainsi être pourvu d'un collecteur d'alimentation commun 17 qui assure d'une part la communication fluidique entre la première et la seconde chambre de serrage 12, 13, et d'autre part la communication entre lesdites chambres de serrage 12, 13 et un dispositif de transfert de fluide externe à l'anneau, de type cathéter (non représenté), par exemple au moyen d'un embout de connexion approprié 18.

Selon une variante de réalisation non représentée, le collecteur d'alimentation commun peut être déporté et comprendre un cathéter commun formant une conduite principale unique, avec lequel communiquent une pluralité de cathéters secondaires débouchant chacun dans une chambre de serrage 12, 13. En particulier, dans le cas où l'anneau comporte deux chambres, le collecteur pourra se présenter sous la forme d'un raccord en Y.

Selon une autre variante de réalisation illustrée sur les figures 4 et 5, le collecteur d'alimentation commun 17 peut être intégré ou accolé à l'anneau. En particulier, ledit collecteur 17 peut être formé par un renflement radial externe qui aborde tangentiellement la portion dorsale de l'anneau 1, sensiblement au niveau du plan sagittal dudit anneau, et qui s'ouvre de manière sensiblement symétrique sur chacune des première et seconde chambres de serrage 12, 13 au moyen d'ouïes 19 spécialement aménagées à cet effet.

Un tel agencement particulièrement simple et compact permet une répartition sensiblement égale et rapide du fluide de remplissage au sein desdites première et seconde chambres de serrage, notamment lors du gonflage initial de ces dernières.

Par ailleurs, selon une variante de réalisation non représentée, les première et seconde chambres de serrage 12, 13 peuvent être sensiblement dépourvues de moyen de communication fluidique entre elles, c'est-à-dire se trouver sensiblement isolée l'une de l'autre de telle sorte qu'aucun transfert de fluide significatif ne puisse s'opérer de la première vers la seconde chambre de serrage, et inversement, de la seconde vers la première chambre de serrage, dans le cadre du fonctionnement normal du dispositif.

En particulier, lesdites première et seconde chambres de serrage 12, 13 peuvent alors être totalement isolées l'une de l'autre, de manière étanche, sans aucune possibilité d'échanger du fluide de remplissage, directement entre elles ou indirectement par leurs systèmes d'alimentation respectifs (cathéter, site implantable, pompe, etc.), ces derniers devant être alors aménagés et séparés en conséquence.

Toutefois, il n'est pas exclu, au sens de l'invention, que le ou les organes de séparation éventuellement disposés entre lesdites première et seconde chambres de serrage 12, 13 présentent une certaine porosité, de telle sorte qu'un léger transfert de fluide de très faible débit, notamment de type suintement, puisse néanmoins se produire entre lesdites chambres, notamment lorsqu'une surpression affecte durablement l'intérieur de l'une desdites chambres.

Cependant, même si un tel fonctionnement par transfert « *amorti »* est envisageable sans sortir du cadre de l'invention, il sera de préférence sensiblement symétrique, c'est-à-dire affectera de manière sensiblement équivalente les transferts de fluide réalisés depuis la première chambre vers la seconde chambre et les transferts réalisés de la seconde chambre vers la première.

De manière plus générale, et quelle que soit la nature du système de communication ou d'isolement retenu, ce dernier sera de préférence sensiblement équilibré, c'est-à-dire n'aura pas tendance à favoriser le transfert de fluide de remplissage de la première chambre vers la seconde, ou de la seconde chambre vers la première, c'est-à-dire le transfert de fluide vers l'une des chambres au détriment de l'autre chambre.

En particulier, ledit système de communication ou d'isolement pourra avantageusement être dimensionné pour ne pas causer de déséquilibre durable lié à une rétention de fluide dans l'une des chambres de serrage 12, 13 et accompagné d'un appauvrissement durable en fluide dans l'autre chambre. En d'autres termes, le système de gestion de fluide mis en oeuvre au sein de l'anneau 1 conforme à l'invention ne sera de préférence pas de nature à provoquer le relâchement durable de l'effort de serrage exercé par l'une ou l'autre des chambres, c'est-à-dire ne sera pas de nature à priver durablement l'anneau de l'un ou l'autre de ses points d'appui, et permettra un maintien sensiblement simultané et permanent des efforts d'appui dans les deux zones de contact 14A, 14B correspondantes.

Par ailleurs, selon une autre caractéristique de l'invention, les première et seconde chambres de serrage 12, 13 sont avantageusement attachées l'une à l'autre.

Ainsi, lesdites première et seconde chambres de serrage 12, 13 sont liées cinématiquement, et plus particulièrement sont solidaires au moins selon l'axe moyen d'extension (XX'), de telle sorte que chaque chambre de serrage est susceptible de retenir l'autre lorsque cette dernière tend à glisser ou à rouler le long de la paroi stomacale. En d'autres termes, la redondance des chambres de serrage permet d'assurer une retenue mutuelle desdites chambres, et sécurise ainsi le maintien de l'anneau 1 dans son ensemble.

A cet effet, lesdites première et seconde chambres de serrage 12, 13 pourront être liées l'une à l'autre à l'aide de tout moyen de connexion 15 approprié, et notamment par des filins, des tiges, des bandelettes, un treillis, etc.

Toutefois, de façon préférentielle, l'anneau chirurgical 1 comporte une bande souple 16 dont la longueur correspond sensiblement au périmètre de la boucle 2, la première et la seconde chambre de serrage 12, 13 étant fixées à ladite bande souple 16 de sorte à faire saillie sur la face interne 16I de cette dernière.

De préférence les chambres de serrage 12, 13 s'étendent sensiblement sur toute la longueur de ladite bande souple 16, et de façon particulièrement préférentielle de façon continue. Bien entendu, il est également envisageable que les chambres de serrage 12, 13 *« annulaires »* ne couvrent qu'une portion de ladite bande souple, c'est-à-dire soient l'une et/ou l'autre fractionnées dans le sens de leur longueur par des zones d'interruption plus ou moins étendues et/ou présentent une couverture angulaire inférieure à 360° autour de l'axe moyen d'extension (XX').

Avantageusement, le moyen de connexion 15, et en particulier la bande souple 16, pourra former la paroi dorsale de l'anneau 1, et notamment délimiter les première et seconde chambres de serrage 12, 13 sensiblement à l'opposé de leurs zones de contact 14A, 14B respectives avec l'organe à enserrer 3, tel que cela est illustré sur les figures.

Selon une variante de réalisation, la bande souple 16 pourra également présenter une certaine mémoire de forme de sorte à adopter spontanément, dans sont état de repos, une forme incurvée tendant à « *amorcer »* la boucle 2, tel que cela est illustré sur la figure 1.

Bien entendu, ladite bande souple est suffisamment souple pour fléchir afin de conférer à la boucle 2 sa courbure autour de l'axe d'extension (XX').

En revanche, selon une variante préférentielle de réalisation, elle présentera une certaine résistance à l'extension en traction, parallèlement à l'axe moyen d'extension (XX'), de sorte à empêcher sensiblement la première chambre de serrage 12 de s'éloigner de la seconde chambre de serrage 13 selon cette direction, et réciproquement.

Selon une variante alternative (non représentée), ladite bande souple 16, et plus globalement le moyen de connexion 15, pourra au contraire être pourvue de moyens d'adaptation axiale conçus pour autoriser un certain débattement axial relatif des chambres de serrage 12, 13 dans des limites prédéfinies par construction.

Une telle disposition constructive permet avantageusement à l'anneau d'adopter un comportement particulièrement souple lui permettant d'accompagner sensiblement les mouvements, notamment péristaltiques, de l'estomac selon leur composante axiale. Ceci permet à l'anneau de conserver une bonne assise sans pour autant créer une sensation prononcée de gêne au patient, ni provoquer d'abrasion de la paroi stomacale.

A cet effet, les moyens d'adaptation axiale pourront par exemple être pourvus d'organes d'articulation élastiques susceptibles de se déformer pour *« absorber »* une extension (sous traction) ou une contraction (sous compression) axiale de l'anneau 1, ou encore comporter des zones de déformation préférentielle assouplies par enlèvement de matière, telles que des entailles ou des lumières ménagées dans la bande souple 16.

Par ailleurs, selon une variante de réalisation préférentielle, l'anneau chirurgical 1 présente, entre la première chambre de serrage 12 et la seconde chambre de serrage 13, une zone de dégagement 20 vide de matière.

Avantageusement, une telle disposition constructive permet de limiter sensiblement le contact effectif entre l'organe 3 et l'anneau 1 aux seules zones de contact 14A, 14B qui correspondent aux crêtes des chambres de serrage 12, 13 faisant saillie sur la face interne de l'anneau 1.

En outre, cette disposition constructive présente plusieurs avantages fonctionnels.

D'une part elle confère à l'anneau 1 un caractère sensiblement atraumatique, dans la mesure où elle limite l'étendue du contact entre l'organe 3 et l'anneau 1 aux seules surfaces fonctionnelles utiles 14A, 14B de ce dernier, et par conséquent réduit considérablement les risques de nécrose ou d'irritation de la paroi stomacale.

En particulier, la paroi stomacale peut ainsi évoluer relativement librement dans la zone de dégagement 20, et notamment présenter un bombé entre la première et la seconde chambre de serrage sans pour autant venir au contact d'une quelconque portion de l'anneau, ce qui évite de meurtrir ladite paroi stomacale.

D'autre part, la zone de dégagement 20 permet de marquer une transition entre la première chambre de serrage 12 et la seconde chambre de serrage 13, laquelle forme une frontière d'isolement mécanique du fait de la discontinuité du contact entre la membrane stomacale et l'anneau au niveau de ladite zone de dégagement 20.

Cette discontinuité spatiale entre les deux zones de contact 14A, 14B confère avantageusement un comportement indépendant de l'anneau dans chacune de ces zones de contact 14A, 14B, et plus précisément évite qu'un glissement dudit anneau 1 par rupture du contact dans la première zone de contact 14A n'entraîne un glissement global de l'anneau, par prolongement continu des conditions d'apparition d'un tel phénomène dynamique, au niveau de la seconde zone de contact 14B.

De préférence, la zone de dégagement 20 s'étend à cet effet sensiblement sur tout le périmètre de la boucle 2, de telle sorte que, une fois l'anneau 1 implanté, les zones de contact 14A, 14B respectives entre l'organe 3 et les première et seconde chambres de serrage 12, 13 sont disjointes.

Ainsi, en séparant complètement les zones de contact 14A, 14B, l'anneau 1 conforme à l'invention présente deux lignes, ou plus précisément deux bandes de contact 14A, 14B distinctes et séparées, et de préférence relativement étroites, sur lesquelles se répartissent les appuis.

L'anneau conforme à l'invention se distingue ainsi des anneaux de l'art antérieur qui, quelle que soit par ailleurs l'épaisseur de leur chambre de serrage (mesurée selon l'axe moyen d'extension (XX')), présentent une bande de contact unique formant une prise continue et localisée sur l'organe 3, et sont par conséquents sujets au glissement ou au retournement dès lors que le contact est rompu au niveau de cette bande de contact unique, même si celle-ci est relativement large.

Au contraire, au sein de l'anneau 1 conforme à l'invention, la zone de dégagement évite la continuité entre les zones de contact 14A, 14B, ce qui limite la propagation des phénomènes de glissement relatifs de l'anneau 1 par rapport à la paroi stomacale.

De même, on réduit considérablement les risques d'inversion de l'anneau par rotation sur lui-même autour de la ligne moyenne qui suit le périmètre de la boucle 2.

En effet, même à supposer que la paroi stomacale tendrait à *« fuir »* hors de l'anneau 1 selon la direction (XX'), et tendrait par conséquent à faire basculer sur elle-même la première chambre de serrage 12 en entraînant cette dernière par friction selon un effet de pelage, on comprend aisément que la rupture de contact induite par la zone de dégagement 20 entre la paroi stomacale et la paroi interne de l'anneau empêche la propagation continue de cet effet de pelage.

Au contraire, dès lors que le moyen de connexion 15 présente une certaine rigidité vis-à-vis d'une flexion autour d'un axe compris dans un plan normal à l'axe moyen d'extension (XX') et sensiblement tangent à la dorsale de l'anneau, le *« pelage inteme »* de la première chambre de serrage 12 peut favoriser l'apparition d'un phénomène de levier de type arc-boutement par lequel la seconde chambre de serrage 13 tend à conforter son appui sur l'organe 3.

De préférence, la zone de dégagement 20 comprend une dépression 21 ménagée sur la face interne 161 de la bande souple 16 et dont le fond est placé en retrait par rapport aux première et seconde chambres de serrage 12, 13, ce qui permet avantageusement à l'anneau de rester à distance de la paroi de l'organe 3 dans ladite zone de dégagement 20, voire sensiblement hors de portée dudit organe 3, lorsque ledit anneau est implanté, et les chambres de serrage gonflées.

Ainsi, tel que cela est illustré notamment sur les figures 3 et 4, la zone de dégagement 20 peut se présenter sous la forme d'une tranchée ou d'une entaille marquée en creux entre lesdites première et seconde chambres de serrage 12, 13, le fond de ladite tranchée ou entaille étant situé à une distance plus importante de l'axe moyen d'extension (XX') que les crêtes desdites chambres de serrage 12, 13 formant les zones de contact 14A, 14B avec l'organe 3.

Avantageusement, la dépression 21 est suffisamment étendue, et notamment suffisamment profonde, c'est-à-dire le décrochement suffisamment marqué, pour éviter la continuité entre lesdites surfaces de contact 14A, 14B, l'évidement ainsi réalisé permettant à cette discontinuité de subsister sensiblement en permanence, quel que soit le comportement de l'organe 3 et l'action de ce dernier sur l'anneau 1.

Par ailleurs, l'anneau 1 conforme à l'invention comporte de préférence un élément anti-rapprochement 22 disposé entre la première et la seconde chambre de serrage 12, 13 et conçu pour maintenir un écartement minimal prédéterminé entre ces dernières.

Plus particulièrement, cet élément anti-rapprochement 22 permet de conserver une largeur minimale de la dépression 21, c'est-à-dire de maintenir la première et la seconde chambre de serrage 12, 13 à distance l'une de l'autre en s'opposant, au moins en partie, à un tassement de l'anneau par compression selon l'axe moyen d'extension (XX').

Avantageusement, on évite ainsi toute apparition, même accidentelle, d'une continuité entre les zones de contact 14A, 14B.

L'élément anti-rapprochement 22 peut notamment être formé par une entretoise semi-rigide ou tout autre moyen équivalent. De préférence, ledit élément anti-rapprochement 22 est formé par une portion renforcée de la bande souple 16, par exemple une surépaisseur dorsale et/ou une ou plusieurs nervures.

Il est par ailleurs remarquable que, si le moyen de connexion 15, et plus particulièrement la bande souple 16, peut, selon une variante de réalisation, permettre le maintien d'une distance axiale sensiblement fixe entre les première et seconde chambres de serrage 12, 13, en s'opposant à la fois à leur éloignement et à leur rapprochement selon cet axe, ledit moyen de connexion 15 peut avantageusement présenter une certaine souplesse radiale, de sorte à autoriser un certain débattement relatif desdites première et seconde chambres de serrage 12, 13 selon des directions transverses à l'axe d'extension (XX').

Ainsi, en évitant de corseter de manière rigide l'estomac dudit patient sur un tronçon de longueur relativement importante, l'anneau conforme à l'invention ne crée pas de gêne significative pour ledit patient.

Bien entendu, l'élément anti-rapprochement 22 peut indifféremment être adapté pour maintenir une distance axiale fixe entre les chambres de serrage 12, 13, ou encore pour fixer un seuil minimal d'écartement résiduel dans le cas ou le moyen de connexion autorise un certain rapprochement relatif desdites chambres de serrage.

Selon une variante de réalisation préférentielle, la première et la seconde chambres de serrage 12, 13 sont délimitées par une membrane commune 30 destinée à être en contact avec l'organe 3 à enserrer.

En d'autres termes, les parois internes desdites première et seconde chambres de serrage 12, 13 destinées à venir au contact de l'organe 3 sont avantageusement venues de matière l'une avec l'autre.

De préférence, ladite membrane commune 30 est réalisée dans un matériau élastomère, présente une épaisseur sensiblement constante, de préférence inférieure à celle de la bande souple 16 formant la face dorsale de l'anneau 1.

De préférence, la membrane commune 30 est en outre accolée, sur au moins une partie de sa longueur, contre la face interne 161 de la bande souple 16 pour former la zone de dégagement 20.

En d'autres termes, les première et seconde chambres de serrage 12, 13 sont avantageusement formées en venant cloisonner par écrasement en son milieu une chambre initialement unique délimitée d'une part par la membrane commune 30 formant sa paroi interne et d'autre part par la bande souple 16 formant sa paroi dorsale.

Plus particulièrement, tel que cela est illustré sur la figure 4, la membrane commune 30, considérée dans sa section transversale, pourra présenter in *fine* un profil comprenant deux dômes voûtés 31, 32 faisant saillie vers l'intérieur de l'anneau 1 et décollés de la face interne 161 de la bande souple 16, lesdits dômes étant séparés par un tronçon intermédiaire 33 proche de ladite face interne 16I de la bande souple, et de préférence collé de manière étanche à cette dernière.

En d'autres termes, les chambres de serrage 12, 13 peuvent avantageusement former, au moins lorsqu'elles sont remplies de fluide, deux boudins annulaires faisant saillie vers l'intérieur de l'anneau, lesdits boudins étant empilés et séparés l'un de l'autre par une zone de transition formant un renfoncement qui peut constituer la zone de dégagement.

Pour parfaire la fixation, la membrane commune 30 pourra également se prolonger par un ou plusieurs rabats 34, 35 assurant son maintien contre la face dorsale de la bande souple 16.

Selon une variante de réalisation préférentielle, la portion de la membrane commune 30 correspondant à la zone de dégagement 20 sera accolée contre la face interne 16I de la bande souple sensiblement sur tout le périmètre de la boucle 2, c'est-à-dire sensiblement sur toute la longueur de ladite bande souple 16, à l'exception de la portion située en vis-à-vis du collecteur d'alimentation commun 17, dans laquelle un léger décollement entre la membrane commune 30 et la bande souple 16 permet avantageusement au fluide de remplissage de circuler entre les chambres de serrage 12, 13 via ledit collecteur.

Il est remarquable que le recours à une membrane commune 30 associée à une même bande souple 16 permet de réaliser une structure simple et légère tout en garantissant une étanchéité optimale des chambres de serrage 12, 13.

Selon une autre variante de réalisation (non représentée), la première et la seconde chambres de serrage 12, 13 sont réalisées séparément, puis rapportées chacune à leur tour sur la bande souple 16, de préférence par collage. Chaque chambre peut par exemple être formée en enroulant sur elle-même une membrane en élastomère de sorte à créer une poche tubulaire individuelle.

Par ailleurs, tel que cela est représenté sur les figures, la première et la seconde chambres de serrage 12, 13 s'étendent sensiblement parallèlement l'une à l'autre, de façon sensiblement normale au même axe d'extension (XX') rectiligne.

En outre, lesdites chambres de serrage 12, 13 présentent de préférence des formes et des dimensions sensiblement identiques, de telle sorte que l'anneau conforme à l'invention possède une symétrie sagittale par rapport à un plan sensiblement normal à l'axe d'extension (XX') et situé à mi-distance entre les deux chambres de serrage 12, 13.

Bien entendu, il est parfaitement envisageable, sans sortir du cadre de l'invention, de disposer plus de deux chambres de serrage étagées, de sorte à former globalement un anneau correspondant à un empilement de plusieurs anneaux élémentaires, de préférence séparés deux à deux par des zones de dégagement et de façon particulièrement préférentielle tous reliés par un même circuit d'alimentation en fluide de remplissage commun.

Bien entendu, l'homme du métier sera également à même d'apprécier les dimensions idoines de l'anneau 1, des chambres de serrage 12, 13 et de la zone de dégagement 20 en fonction de l'application visée.

En particulier, il n'est pas exclu de prévoir des chambres de serrage de dimensions différentes, et notamment de volumes et/ou de diamètres rentrants distincts, de telle sorte que l'anneau 1 présente un étranglement plus prononcé à certains étages qu'à d'autres.

En outre, selon une variante de réalisation qui peut constituer une invention en tant que telle, l'anneau 1 peut comporter une pluralité de chambres de serrage annulaire, et notamment au moins trois chambres, empilées axialement et sensiblement accolées les unes aux autres à distance de leurs zones de contact respectives avec la paroi stomacale, de manière à former une sorte de *« chenille »* ou de manchon adaptable constitué d'anneaux élémentaires articulés les uns par rapport aux autres, permettant ainsi à l'anneau gastrique d'épouser dans son ensemble la forme de l'estomac en toutes circonstances.

A titre purement indicatif, un anneau gastrique conforme à l'invention pourra présenter les dimensions suivantes :
- Epaisseur mesurée entre les deux bords latéraux de l'anneau selon l'axe moyen d'extension (XX') sensiblement comprise entre 10 mm et 30 mm.
- Diamètre de serrage nominal (c'est-à-dire diamètre des portions rentrantes des chambres de serrage lorsque celles-ci sont gonflées, mesuré transversalement à l'axe moyen d'extension (XX')) sensiblement compris entre 8 mm et 30 mm.
- Diamètre de dégagement (mesuré transversalement à l'axe d'extension (XX') au niveau du fond de la dépression 21) sensiblement compris entre 30 mm et 35 mm.
- Epaisseur de chaque chambre (largeur de base de la section transversale de la cavité, mesurée parallèlement à l'axe d'extension (XX') sensiblement comprise entre 4 mm et 12 mm.
- Hauteur de dégagement (largeur de la dépression 21 mesurée parallèlement à l'axe d'extension (XX') et correspondant à la distance de séparation entre la première et la seconde chambre) sensiblement comprise entre 0 mm (chambres accolées par leur base) et 10 mm.

Par ailleurs, la présente invention concerne également un procédé de fabrication d'un anneau chirurgical implantable 1 tel que décrit précédemment, et plus précisément d'un anneau chirurgical implantable destiné à être fermé sur lui-même pour former une boucle close d'axe moyen d'extension (XX') afin d'enserrer un organe biologique 3 constituant une poche ou un conduit en vue de modifier la section de passage dudit organe.

Selon l'invention, ledit procédé comporte une étape (a) de préparation au cours de laquelle on réalise au moins une première chambre de serrage 12 annulaire et une seconde chambre de serrage 13 annulaire étant chacune destinée à contenir un fluide de remplissage.

Une telle étape de préparation peut notamment comprendre une sous-étape de mise en forme, notamment par injection ou moulage d'une ou plusieurs membranes en élastomère destinées à délimiter lesdites chambres de serrage.

En outre, le procédé conforme à l'invention comporte une étape (b) d'étagement au cours de laquelle on relie mécaniquement lesdites première et seconde chambres de serrage l'une à l'autre en les disposant de manière étagées l'une par rapport à l'autre le long de l'axe moyen d'extension (XX') de la boucle afin que l'anneau 1 puisse serrer l'organe 3 au niveau d'au moins deux zones de contact 14A, 14B séparées axialement.

Plus particulièrement, ladite étape (b) d'étagement peut comprendre une sous-étape (b1) d'assemblage au cours de laquelle on fixe la première et la seconde chambre de serrage 12, 13 sur la face interne d'une même bande souple 16, ainsi qu'une sous-étape (b2) de délimitation au cours de laquelle on ménage une zone de dégagement 20 vide de matière entre lesdites première et seconde chambres de serrage 12, 13.

De façon particulièrement préférentielle, la sous-étape (b2) de délimitation comprend une phase de jonction en creux au cours de laquelle on rapporte en vis-à-vis de la face interne 161 de la bande souple 16 une membrane 30 commune aux première et seconde chambres de serrage 12, 13, puis l'on plaque la portion de ladite membrane commune 30 correspondant à la zone de dégagement 20 contre ladite bande souple 16 de sorte à la faire adhérer à cette dernière.

De façon particulièrement avantageuse, cette opération peut être mise en oeuvre à plat, c'est-à-dire alors que la bande souple 16 est déroulée, au moyen d'un procédé d'estampage ou de laminage au cours duquel un poinçon ou une molette vient écraser la partie centrale de la membrane commune 30, de sorte à cloisonner ce qui était au départ une poche unique et commune en deux chambres de serrage 12, 13 distinctes.

Avantageusement, cette phase de jonction en creux est mise en oeuvre seulement sur une portion du pourtour de la boucle 2, c'est-à-dire de la longueur de la bande souple 16, de manière à préserver au moins une zone de libre communication entre les première et seconde chambres de serrage 12, 13, de préférence en vis-à-vis du collecteur d'alimentation 17.

Enfin, le procédé conforme à l'invention comprend également une étape (c) d'agencement fluidique au cours de laquelle soit on ménage une communication fluidique bidirectionnelle libre entre les première et seconde chambres de serrage 12, 13, soit on isole sensiblement lesdites chambres l'une de l'autre.

Plus particulièrement, la réalisation d'une communication fluidique bidirectionnelle libre peut être réalisée en préservant ou en creusant un ou plusieurs orifices de communication entre lesdites chambres. A l'inverse, on peut également isoler sensiblement lesdites chambres, soit de manière étanche au moyen d'une ou plusieurs parois éventuellement distinctes, soit encore par une cloison commune présentant éventuellement une légère porosité.

Ainsi, le procédé de fabrication conforme à l'invention permet de réaliser de manière particulièrement simple, rapide et peu onéreuse un anneau chirurgical présentant une excellente stabilité une fois implanté.

En outre, un tel anneau conforme à l'invention limite considérablement le traumatisme subi par le patient et améliore le confort et la fiabilité du traitement sur le long terme.

Enfin, la structure même de l'anneau conforme à l'invention lui confère une légèreté, une compacité et une simplicité de mise en oeuvre particulièrement appréciable tant par le praticien que par le patient lui-même.

### POSSIBILITE D'APPLICATION INDUSTRIELLE

L'invention trouve son application industrielle dans la conception et la fabrication de dispositifs médicaux implantables notamment destinés au traitement de l'obésité.

## Revendications

1. Anneau chirurgical implantable (1) destiné à être fermé sur lui-même pour former une boucle close (2) d'axe moyen d'extension (XX') afin d'enserrer un organe biologique (3) constituant une poche ou un conduit en vue de modifier la section de passage dudit organe (3), ledit anneau (1) comportant au moins une première chambre de serrage (12) annulaire et une seconde chambre de serrage (13) annulaire attachées l'une à l'autre et destinées à contenir un fluide de remplissage pour assurer le serrage dudit organe biologique (3) par ledit anneau, lesdites première et seconde chambres de serrage étant étagées l'une par rapport à l'autre le long de l'axe moyen d'extension (XX') de la boucle (2), l'anneau comportant une bande souple (16) dont la longueur correspond sensiblement au périmètre de la boucle (2), la première et la seconde chambres de serrage (12, 13) sont fixées à ladite bande souple (16) de sorte à faire saillie sur la face interne (161) de cette dernière, ledit anneau étant **caractérisé en ce que** la première et la seconde chambres de serrage (12, 13) gonflées présentent entre elles une zone de dégagement (20) vide de matière, dans laquelle la paroi de l'organe biologique (3) peut évoluer sans venir au contact de l'anneau (1), pour assurer le serrage dudit organe biologique (3) au niveau d'au moins deux zones de contact (14A, 14B) séparées axialement.

2. Anneau chirurgical selon la revendication 1 **caractérisé en ce que** la zone de dégagement (20) s'étend sensiblement sur tout le périmètre de la boucle (2) de telle sorte que, une fois l'anneau implanté, les zones de contact (14A, 14B) respectives entre l'organe biologique (3) et les première et seconde chambres de serrage (12, 13) sont disjointes.

3. Anneau chirurgical selon l'une des revendications 1 ou 2 **caractérisé en ce qu'**il comporte un élément anti-rapprochement (22) disposé entre la première et la seconde chambre de serrage (12, 13) et conçu pour maintenir un écartement minimal prédéterminé entre ces dernières.

4. Anneau chirurgical selon l'une quelconque des revendications précédentes, **caractérisé en ce que** la zone de dégagement (20) comprend une dépression (21) ménagée sur la face interne (16l) de la bande souple (16) et dont le fond est placé en retrait par rapport aux première et seconde chambres de serrage (12, 13).

5. Anneau chirurgical selon la revendication 3, **caractérisé en ce que** l'élément anti-rapprochement (22) est formé par une portion renforcée de la bande souple (16).

6. Anneau chirurgical selon l'une des revendications précédentes **caractérisé en ce qu'**il est pourvu de moyens d'adaptation axiale conçus pour autoriser le débattement axial relatif des chambres de serrage (12, 13) dans des limites prédéfinies par construction.

7. Anneau chirurgical selon l'une des revendications précédentes **caractérisé en ce que** la première et la seconde chambres de serrage (12, 13) s'étendent sensiblement parallèlement l'une à l'autre.

8. Anneau chirurgical selon l'une des revendications précédentes **caractérisé en ce que** les première et seconde chambres de serrage (12, 13) sont délimitées par une membrane commune (30) destinée à être en contact avec l'organe (3) à enserrer.

9. Anneau chirurgical selon la revendication 8 **caractérisé en ce que** la membrane commune (30) est accolée, sur au moins une partie de sa longueur, contre la face interne (161) de la bande souple (16) pour former la zone de dégagement (20).

10. Anneau chirurgical selon l'une des revendications précédentes **caractérisé en ce que** la première et la seconde poche de serrage (12, 13) se trouvent soit en communication fluidique bidirectionnelle libre, soit sensiblement dépourvues de moyens de communication fluidique entre elles.

11. Anneau chirurgical selon l'une des revendications précédentes **caractérisé en ce qu'**il est pourvu d'un collecteur d'alimentation commun (17) assurant d'une part la communication fluidique entre la première et la seconde chambre de serrage (12, 13), et d'autre part la communication entre lesdites chambres de serrage (12, 13) et un dispositif de transfert de fluide externe à l'anneau, de type cathéter.

12. Anneau chirurgical selon la revendication 11 **caractérisé en ce que** le collecteur d'alimentation commun est intégré à l'anneau et s'ouvre de manière sensiblement symétrique sur chacune des première et seconde chambres de serrage (12, 13).

13. Anneau chirurgical selon l'une des revendications précédentes **caractérisé en ce qu'**il constitue un anneau de gastroplastie.

14. Procédé de fabrication d'un anneau chirurgical implantable (1) destiné à être fermé sur lui-même pour former une boucle close (2) d'axe moyen d'extension (XX') afin d'enserrer un organe biologique (3) constituant une poche ou un conduit en vue de modifier la section de passage dudit organe, ledit procédé comportant une étape (a) de préparation au cours de laquelle on réalise au moins une première chambre de serrage (12) annulaire et une seconde chambre de serrage (13) annulaire étant chacune destinée à contenir un fluide de remplissage, une étape (b) d'étagement au cours de laquelle on relie mécaniquement lesdites première et seconde chambres de serrage (12, 13) l'une à l'autre en les disposant de manière étagée l'une par rapport à l'autre le long de l'axe moyen d'extension (XX') de la boucle (2), l'étape (b) d'étagement comprenant une sous-étape (b1) d'assemblage au cours de laquelle on fixe la première et la seconde chambre de serrage (12, 13) sur la face interne (161) d'une même bande souple (16) ledit procédé étant **caractérisé en ce que** l'étape (b) d'étagement comprend une sous-étape (b2) de délimitation au cours de laquelle on ménage entre lesdites première et seconde chambres de serrage (12, 13) une zone de dégagement (20) vide de matière dans laquelle la paroi de l'organe biologique (3) puisse évoluer sans venir au contact de l'anneau (1) lorsque les première et seconde chambres de serrage sont gonflées, afin que les première et seconde chambres de serrage (12, 13) gonflées puissent serrer l'organe biologique (3) au niveau d'au moins deux zones de contact (14A, 14B) séparées axialement.

15. Procédé de fabrication selon la revendication 14 **caractérisé en ce que** la sous-étape (b2) de délimitation comprend une phase de jonction en creux au cours de laquelle on rapporte en vis-à-vis de la face interne (161) de la bande souple (16) une membrane (30) commune aux première et seconde chambres de serrage (12, 13) puis l'on plaque la portion de ladite membrane commune (30) correspondant à la zone de dégagement (20) contre ladite bande souple (16) de sorte à la faire adhérer à cette dernière.

## Patentansprüche

1. Implantierbarer chirurgischer Ring (1), der sich selbst schließen soll und so eine geschlossene Schlinge (2) mit mittlerer Erweiterungsachse (XX') bildet, um damit ein biologisches Organ (3), das die Form einer Tasche oder eines Durchgangs hat, einzuschnüren, um die Durchgangsbreite dieses Organs (3) zu verändern, dieser Ring (1) umfasst mindestens eine erste ringförmige Klemmkammer (12) und eine zweite ringförmige Klemmkammer (13), die miteinander verbunden sind und eine Füllflüssigkeit aufnehmen sollen, mit der die Einschnürung des biologischen Organs (3) erreicht wird, die beiden Klemmkammern sind auf Höhe der mittlerer Erweiterungsachse (XX') der Schlinge (2) abgestuft angeordnet, der Ring besteht aus einem weichen Band (16), dessen Länge genau dem Umfang der Schlinge (2) entspricht, die erste und die zweite Klemmkammer (12, 13) sind so an diesem weichen Band (16) befestigt, dass sie über dessen Innenseite (16l) hinausragen,
dieser Ring zeichnet sich dadurch aus, dass die erste und die zweite gefüllte Klemmkammer (12, 13) zusammen eine freie, materialleere Zone (20) bilden, in der die Wand des biologischen Organs (3) sich ausbreiten kann, ohne in Kontakt zum Ring (1) zu kommen, um die Einschnürung dieses biologischen Organs (3) auf Höhe mindestens einer der beiden axial getrennten Kontaktzonen (14A, 14B) zu gewährleisten.

2. Der chirurgische Ring ist nach Anspruch 1 **dadurch gekennzeichnet, dass** die freie Zone (20) sich deutlich über den gesamten Umfang der Schlinge (2) erstreckt, so dass nach dem Einsatz des Rings die Kontaktzonen (14A, 14B) jeweils zwischen dem biologischen Organ (3) und der ersten und zweiten Klemmkammer (12, 13) voneinander getrennt sind.

3. Der chirurgische Ring ist nach Anspruch 1 bzw. 2 **dadurch gekennzeichnet, dass** er ein Anti-Annäherungs-Element (22) beinhaltet, das sich zwischen der ersten und der zweiten Klemmkammer befindet (12, 13) und so beschaffen ist, dass es einen Mindestabstand zwischen diesen beiden Kammern aufrechterhält.

4. Der chirurgische Ring ist nach einem beliebigen der vorherigen Ansprüche **dadurch gekennzeichnet, dass** die freie Zone (20) eine Mulde (21) zur Innenseite (16l) des weichen Bands (16) aufweist, und in der Tiefe hinter den ersten und dem zweiten Klemmring (12, 13) geht.

5. Der chirurgische Ring ist nach Anspruch 3 **dadurch gekennzeichnet, dass** das Anti-Annäherungs-Element (22) durch einen verstärkten Teil des weichen Bands (16) gebildet wird.

6. Der chirurgische Ring ist nach einem der vorherigen Ansprüche **dadurch gekennzeichnet, dass** er mit einer axialen Anpassungsmöglichkeit ausgestattet ist, die so beschaffen ist, dass sie die relative axiale Ausweitung der Klemmkammern (12, 13) innerhalb der durch die Konstruktion vorgegebenen Grenzen ermöglicht.

7. Der chirurgische Ring ist nach einem der vorherigen Ansprüche **dadurch gekennzeichnet, dass** die erste und die zweite Klemmkammer (12, 13) sich genau parallel zueinander ausdehnen.

8. Der chirurgische Ring ist nach einem der vorherigen Ansprüche **dadurch gekennzeichnet, dass** die erste und die zweite Klemmkammer (12, 13) durch eine gemeinsame Membran (30) begrenzt sind, die beim Einschnüren mit dem Organ (3) in Kontakt kommen soll.

9. Der chirurgische Ring ist nach Anspruch 8 **dadurch gekennzeichnet, dass** die gemeinsame Membran (30) mindestens über einen Teil ihrer Länge an die Innenseite (16l) des weichen Bands (16) anschließt, um die freie Zone (20) zu bilden.

10. Der chirurgische Ring ist nach einem der vorherigen Ansprüche **dadurch gekennzeichnet, dass** zwischen der ersten und der zweiten Klemmkammer (12, 13) entweder eine freie Flüssigkeitsverbindung besteht, oder dass die Flüssigkeiten beider Kammern deutlich voneinander abgegrenzt sind.

11. Der chirurgische Ring ist nach einem der vorherigen Ansprüche **dadurch gekennzeichnet, dass** er mit einer Versorgungssammelleitung (17) ausgestattet ist, die erstens für eine Flüssigkeitsverbindung zwischen der ersten und der zweiten Klemmkammer (12, 13) sogt und zweites für die Verbindung zwischen diesen Kammern (12, 13), sowie mit einer Flüssigkeitstransfervorrichtung vom Kathetertyp außerhalb des Rings.

12. Der chirurgische Ring ist nach Anspruch 11 **dadurch gekennzeichnet, dass** die Versorgungssammelleitung in den Ring integriert ist und sich genau symmetrisch zur ersten und zur zweiten Klemmkammer (12, 13) hin öffnet.

13. Der chirurgische Ring ist nach einem der vorherigen Ansprüche **dadurch gekennzeichnet, dass** es sich um einen Magenring handelt.

14. Das Herstellungsverfahren eines implantierbaren chirurgischen Rings (1), der sich selbst schließen soll und so eine geschlossene Schlinge (2) mit mittlerer Erweiterungsachse (XX) bildet, um damit ein biologisches Organ (3), das die Form einer Tasche oder eines Durchgangs hat, einzuschnüren, um die Durchgangsbreite dieses Organs zu verändern, besteht aus einem Vorbereitungsschritt (a), bei dem mindestens eine ringförmige Klemmkammer (12) gefertigt wird sowie eine zweite Klemmkammer (13), die jeweils eine Füllflüssigkeit aufnehmen sollen, sowie einem Abstufungsschritt (b), bei dem diese erste und zweite Klemmkammer (12, 13) mechanisch miteinander verbunden werden, indem sie stufenweise zueinander entlang der mittleren Erweiterungsachse (XX') des Rings (2) angeordnet werden. Der Abstufungsschritt (b) beinhaltet dabei einen untergeordneten Montageschritt (b1), bei dem die erste und die zweite Klemmkammer (12, 13) an der Innenseite (16l) eines gemeinsamen weichen Bandes (16) fixiert werden.
Dieses Verfahren ist **dadurch gekennzeichnet, dass** der Abstufungsschritt (b) einen untergeordneten Abgrenzungsschritt (b2) umfasst, bei dem zwischen dieser ersten und zweiten Klemmkammer (12, 13) eine freie, materialleere Zone (20) geschaffen wird, in der die Wand des biologischen Organs (3) sich entwickeln kann, ohne in Kontakt mit dem Ring (1) zu gelangen, wenn die erste und die zweite Klemmkammer gefüllt sind, damit diese gefüllten Klemmkammern (12, 13) das biologische Organ (3) auf Höhe mindestens einer der beiden axial getrennten Kontaktzonen (14A, 14B) einschnüren können.

15. Das Herstellungsverfahren ist nach Anspruch 14 **dadurch gekennzeichnet, dass** der untergeordnete Abgrenzungsschritt (b2) eine Verbindungsphase für den Hohlraum beinhaltet, bei der gegenüber der Innenseite (16l) des weichen Bands (16) eine gemeinsame Membran (30) für die erste und zweite Klemmkammer (12, 13) geschaffen wird und dann der Teil dieser gemeinsamen Membran (30) für die freie Zone (20) so gegen das weiche Band (16) gepresst, dass sie daran haften bleibt.

## Claims

1. Implantable surgical ring (1) intended to be closed on itself to form a closed loop (2) of an extension axis (XX) to clamp a biological organ (3) constituting a pouch or a conduit to modify the passage section of said organ (3), said ring (1) having at least a first annular clamping chamber (12) and a second annular clamping chamber (13) attached to each side and intended to contain a filling fluid for clamping said biological organ (3) by said ring, said first and second clamping chambers being staggered relative to each other along the central axis of extension (XX) of the loop (2), the ring comprising a flexible band (16) whose length corresponds substantially to the perimeter of the loop (2), the first and second clamping chambers (12, 13) being fixed to said flexible strip (16) so as to project on the inner face (161) of the latter, said ring being **characterised in that** the first and second clamping chambers (12, 13) have between them a swollen clearance zone (20) empty of material, wherein the wall of the biological organ (3) can move without coming into contact with the ring (1), to effect clamping of said biological organ (3) in at least two contact areas (14A, 14B) axially separated.

2. Surgical ring according to claim 1 **characterised in that** the clearance (20) extends substantially around the perimeter of the loop (2) such that, once the ring is implanted, the contact areas (14A, 14B) between the respective biological organ (3) and the first and second clamping chambers (12, 13) are disjoint.

3. Surgical ring according to one of claims 1 or 2 **characterised in that** it comprises a strut organ (22) disposed between the first chamber and the second clamp (12, 13) and designed to maintain a predetermined minimum spacing between the latter.

4. Surgical ring according to any one of the preceding claims, **characterised in that** the clearance zone (20) comprises a depression (21) formed on the inner face (161) of the flexible strip (16) and whose bottom is recessed from the first and second clamping chambers (12, 13).

5. Surgical ring according to claim 3, **characterised in that** the strut (22) is formed by a reinforced portion of the flexible band (16).

6. Surgical ring according to one of the preceding claims **characterised in that** it is provided with axial adjustment means adapted to allow relative axial movement of the clamping chambers (12, 13) within limits preset by design.

7. Surgical ring according to one of the preceding claims **characterised in that** the first and second clamping chambers (12, 13) extend substantially parallel to one another.

8. Surgical ring according to one of the preceding claims **characterised in that** the first and second clamping chambers (12, 13) are delimited by a common membrane (30) intended to be in contact with the organ (3) to be clamped.

9. Surgical ring according to claim 8 **characterised in that** the common membrane (30) is contiguous, for at least part of its length, with the inner face (161) of the flexible strip (16) to form the clearance (20).

10. Surgical ring according to one of the preceding claims **characterised in that** the first and second clamping pocket (12, 13) are either in fluid bidirectional free communication or substantially free of means of fluid communication between them.

11. Surgical ring according to one of the preceding claims **characterised in that** it is provided with a common feeding manifold (17) serving firstly for the fluid communication between the first chamber and the second clamp (12, 13), and on secondly for the communication between said clamping chambers (12, 13) and a transfer device to the fluid external ring of the catheter-type.

12. Surgical ring according to claim 11 **characterised in that** the common supply manifold is integrated into the ring and opens substantially symmetrically on each of the first and second clamping chambers (12, 13).

13. Surgical ring according to one of the preceding claims **characterised in that** it constitutes a gastroplasty ring.

14. A method of manufacturing an implantable surgical ring (1) intended to be closed on itself to form a closed loop (2) through an extension axis (XX') in order to clamp a biological organ (3) constituting a pocket or a conduit for modifying the passage section of said organ, said method comprising a step (a) for preparation in which a first annular clamping chamber (12) and a second annular chamber of the clamping is carried out (13) each intended to contain a filling fluid, a staging step (b) in which said first and second clamping chambers (12, 13) are mechanically connected to one another, staggering them with respect to each other along the axis by means of extension (XX') of the loop (2), the staggering step (b) comprising a substep (b1) of assembly in which the first and second clamping chamber (12, 13) are fixed on the inner face (161) of the same flexible strip (16),
said method being **characterised in that** the staggering step (b) comprises a delimitation substep (b2) during which between said first and second clamping chambers (12, 13) a zone of clearance (20) of blank material is arranged in which the wall of the biological organ (3) can move without coming into contact with the ring (1) when the first and second clamping chambers are inflated so that the first and second clamping chambers (12, 13) can clamp the swollen organic organ (3) in at least two contact areas (14A, 14B) axially separated.

15. Production method according to claim 14 **characterised in that** the sub-step (b2) comprises a hollow phase boundary junction in which vis-à-vis the internal face (161) of the flexible strip (16) there is a membrane (30) common to the first and second clamping chambers (12, 13) and the portion of said common membrane (30) corresponding to the clearance (20) against said flexible strip (16) so as to adhere to the latter.
